# EUROPEAN PATENT APPLICATION

(11) **EP 1 125 896 A1**
(43) Date of publication of application: **22.08.2001**
(21) Application number: 00201978.4
(22) Date of filing: 06.06.2000
(51) Int. Cl.: C02F 9/00, A61K 7/15, C11D 17/00

(54) **Method for reducing the emission of unpleasant odors from a water closet and foaming composition for carrying out the same**

(30) Priority: 18.02.2000 IT MI000282
(71) Applicant: Laboratorio Chimico Farmaceutico Sammarinese S.A., 47031 Faetano (SM)
(72) Inventor: Gazzaniga, Giancarlo, 27056 Salice Terme (PV) (IT); Marinelli, Pier Marino, 47899 Serravalle RSM (SM); Gualandi, Michele, 40013 Castelmaggiore (BO) (IT)
(74) Representative: Bottero, Claudio

(57) **Abstract**

The invention relates to a method for reducing the emission of unpleasant odors from a water closet comprising the step of dispensing within the water closet a compact and creamy foam, the foam being such that by dispensing 8 g thereof in a cylindrical container having a diameter of 11 cm, it appears to be essentially constituted by bubbles having a maximum diameter of 1 mm and having a substantially constant volume for at least 10 minutes. The invention also relates to a foaming composition for carrying out said method, as well as to an aerosol bomb provided with dispensing means for delivering a foam from said foaming composition. Advantageously, the foam of the invention acts as a barrier against unpleasant odors possibly released from the water closet.

## Description

### Background of the invention

In a general aspect, the present invention relates to a method for reducing the emission of unpleasant odors from a water closet.

The invention also relates to a foaming composition for carrying out said method, as well as to an aerosol bomb provided with dispensing means for delivering a foam from said foaming composition.

### Prior art

In the field of detergent/perfuming products for toilet appliances, such as for instance water closet bowls, the need has always been felt of reducing as much as possible the impact of unpleasant odors released from the water closet after use of the same.

To this end, compositions either in solid or liquid form are known, which compositions are dispensed in a dosed amount at each flush of flushing water and which carry out both a detergent and sanitizing effect and a deodorizing effect intended to limit to some extent the negative impact of the possible unpleasant odors released from the water closet.

In almost all cases, these compositions show a weak foaming activity essentially associated to the presence of surfactants in their formulation, so that at each discharge of flushing water a short-lasting large-bubble foam is formed which may sometimes prolong the perfuming effect of the composition.

As an alternative or in addition to the use of such compositions, the typical remedy used to limit the impact of possible unpleasant odors provides for dispensing a room deodorant in the toilet after each use of the water closet by means of an aerosol bomb.

While the known products may reduce to some extent the perception of unpleasant odors by, so-to-say, "superposing" on the same, the effect of the dispensed perfume is a short-lasting one, and in some cases entirely insufficient for frequently utilized toilets such as, for instance, public latrines.

### Summary of the invention

The technical problem underlying the present invention is therefore that of devising and providing a method capable to effectively limit in a lasting manner the negative impact of unpleasant odors released from a water closet, also in case of a frequent utilization of the same.

According to the invention, the Applicant has now realized that such problem may be effectively solved not by "superposing" a perfume on the unpleasant odor released from the water closet, but rather by forming a physical barrier capable of mechanically hindering the release of unpleasant odors from the water closet.

Conveniently, this physical barrier is obtained by dispensing within the water closet a creamy, compact, small-bubble foam having as such structural features suitable to effectively hinder the release of unpleasant odors from the water closet.

According to a first aspect of the invention, the above technical problem is therefore solved by a method for reducing the emission of unpleasant odors from a water closet, comprising the step of dispensing within the water closet a compact and creamy foam such that by dispensing 8 g thereof in a cylindrical container having a diameter of 11 cm, the foam is essentially constituted by bubbles having a maximum diameter of 1 mm and a substantially constant volume for at least 10 minutes.

According to the invention, the Applicant has found that the negative impact of unpleasant odors released from a water closet may be effectively reduced by forming within the water closet a mechanical barrier essentially constituted by a foam layer having special features of structure and constitution.

With respect to the foams which are usually generated by the detergent and sanitizing compositions of the prior art, generally formed by large short-lasting bubbles, the foam dispensed in the method of the invention is not only markedly more compact and, as such, suitable to develop the desired "barrier" effect against unpleasant odors, but also more lasting in the course of time, so as to maintain for as long as possible such effect.

More particularly, the foam utilized in the method according to the invention has - after dispensing 8 g thereof in a cylindrical container having a diameter of 11 cm - the following features:
- aspect: compact and creamy foam essentially constituted by bubbles having a maximum diameter of 1 mm,
- volume: substantially constant for at least 10 minutes.

Preferably, the foam formed in this way within said container remains compact and creamy and its volume is not lower than 90% of its starting volume after 30 minutes and not lower than 50% of its starting volume after 60 minutes from its dispensing.

Still more preferably, the foam has a substantially constant volume for at least 30 minutes after its dispensing and slowly dissolves in the course of a time span not shorter than 120 minutes.

In an embodiment of the method according to the invention, the foam is dispensed on the free surface of the stagnant water present in the water closet and optionally on the inner walls of the same near the free surface of the stagnant water and which may get in touch with feces or other solid residues discharged in the water closet.

In the following description and in the appended claims, the expression: stagnant water, is used to indicate the water which is generally present between the water closet bowl and the trap positioned downstream of the same and having the function of sealing in an air-tight manner the water closet from the sewage system.

In a further embodiment of the method according to the invention, applicable when no stagnant water is present in the water closet, such as for instance in toilets of movable vehicles such as campers, caravans, trains and the like, the foam is dispensed on a closing door of a discharge opening of the water closet and optionally on the inner walls thereof near said door.

Conveniently, the method of the invention may provide - in both of the above described embodiments - for a foam dispensing before and/or after the use of the water closet, so as to form a foam layer having an adequate thickness.

For the purposes of the invention, it has been found that a foam layer having a thickness comprised between 10 and 15 cm may effectively develop the desired barrier effect against unpleasant odors.

Preferably, foam dispensing is carried out by hand using a suitable dispensing container such as for instance an aerosol bomb.

Advantageously, the preventive foam dispensing within the water closet before using the same allows to hinder feces from sticking to the walls of the water closet that may get in touch with the same, thanks to the mechanical and lubricating effect carried out by the small-bubble foam of the invention.

In this way, it is possible not only to improve the hygienic conditions of the water closet, especially in case of frequent utilization, as happens for instance in public facilities, but also to avoid to use the brush to the advantage of the practicality of use of the water closet itself.

Within the scope of the invention, a compact, creamy and stable small-bubble foam may be obtained from a range of adequately formulated foaming compositions including: i) a liquid base comprising at least one surfactant having a high foaming capacity and at least one foam stabilizing agent carried by a suitable solvent, and ii) a gaseous propellant, and having a suitable ratio between said liquid base and the gaseous propellant.

More particularly, the Applicant has found that the above identified technical problem may be effectively solved by a foaming composition capable to generate a foam adapted to reduce the emission of unpleasant odors from a water closet comprising:
a) from 70 to 95% by weight of a liquid base including:
a1) from 50 to 90% by weight based on the total weigh of the liquid base of a suitable solvent;
a2) from 0.2 to 5% by weight based on the total weigh of the liquid base of a nonionic ethoxylated surfactant selected from ethoxylated alcohols having the formula

   CH₃(CH₂)ₙCH₂(OCH₂CH₂)ₘOH

   wherein n is an integer of from 10 to 16, m is an integer of from 2 to 24, salts of said alcohols soluble in said solvent, and mixtures thereof,
a3) from 2 to 12% by weight based on the total weight of the liquid base of a foam stabilizing agent,
   the weight ratio between said nonionic ethoxylated surfactant and said foam stabilizing agent being comprised between 5:1 and 2:1, and
b) from 5 to 30% by weight of a gaseous propellant.

According to the invention, such a foaming composition may be usefully employed for carrying out the above described method.

To this aim, it may be dispensed from an aerosol bomb comprising:
- a pressurized container including a dosed amount of foaming composition, and
- dispensing means for delivering a foam from said foaming composition.

Preferably, the solvent is present in the liquid base in a quantity comprised between 70 and 90% by weight and, still more preferably, between 75 and 80% by weight based on the total weight of the same.

For the purposes of the invention, demineralized water is the solvent of preferred and most advantageous use for the formation of a suitable liquid base.

In the foaming composition of the invention, the nonionic ethoxylated surfactant carries out the important function of generating a foam, when the composition is dispensed and in combination with the solvent, thanks to the known modifying action of surfactants of the solvent surface tension.

Preferably, the liquid base comprises a quantity of nonionic ethoxylated surfactant comprised between 1 and 2.5% by weight based on the total weight of the same.

In a preferred embodiment, the nonionic ethoxylated surfactant is essentially constituted by a mixture of water-soluble alcohols and specifically by a mixture of polyoxyethylene(20)cetyl alcohol and polyoxyethylene(4)lauryl alcohol.

The Applicant has in fact found that thanks to the use of such a mixture, it is advantageously possible to obtain an optimal reduction of the surface tension and a correct ratio of the hydrophilic to the hydrophobic portions or HLB (Hydrophilic/Lipophilic Balance) of the surfactant which contributes to increasing the foam duration within the aforementioned range of values.

Preferably, the nonionic ethoxylated surfactant comprises:
- from 0.5 to 3% by weight of polyoxyethylene(20)cetyl alcohol,
- from 0.2 to 2% by weight of polyoxyethylene(4)lauryl alcohol.

Still more preferably, the nonionic ethoxylated surfactant comprises:
- from 0.8 to 1.2% by weight of polyoxyethylene(20)cetyl alcohol,
- from 0.6 to 1% by weight of polyoxyethylene(4)lauryl alcohol.

Preferably, the weight ratio between the polyoxyethylene(20)cetyl alcohol and the polyoxyethylene(4)lauryl alcohol is comprised between 0.8 and 2.0.

By observing such ratios it was found that it is possible to achieve an optimal foam duration within the aforesaid range of values.

Preferably, the aforementioned stabilizing agent is present in the liquid base in a quantity comprised between 4 and 10% by weight and, still more preferably, between 5 and 8% by weight based on the total weight of the same.

In the foaming composition of the invention, the stabilizing agent carries out the important function of contributing to render the foam compact and stable in the course of time.

To this aim, the Applicant has found that, in order to obtain an adequate effect of foam stabilization, the weight ratio of said nonionic ethoxylated surfactant to said foam stabilizing agent should be comprised in the range between 5:1 and 2:1 and, more preferably, between 3:1 and 4:1.

Preferably, the foam stabilizing agent is selected from amine alkyl oxides, coconut monoethanolamide, sodium lauryl succinate and polymeric stabilizing agents, among which polyoxyethyleneglycol, hydrocolloids, optionally functionalized with a quaternary ammonium group, acrylic and methacrylic polymers, and mixtures thereof.

In the following description and in the appended claims, the term: hydrocolloids, is used to indicate natural or synthetic polymers capable of absorbing the employed solvent, originating a colloidal solution capable of stabilizing the produced foam.

For the purposes of the invention, the hydrocolloids of most advantageous and preferred use are selected from the group comprising: carragenin, xanthane gum, guar gum, cellulose derivatives, optionally functionalized with a quaternary ammonium group, and mixtures thereof.

In a preferred embodiment, the foam stabilizing agent is a polyoxyethyleneglycol having a molecular weight comprised in the range between 5400 and 6600, which was found to perform an optimal effect of foam stabilization.

In a further embodiment, the foam stabilizing agent comprises one or more hydrocolloids in combination with at least one additional stabilizing agent.

In this case, it is preferred and advantageous to employ the hydrocolloid(s) in a quantity ranging between 0.2 and 2% by weight based on the total weight of the liquid base, the balance used to obtain the desired total amount of stabilizing agent being constituted by the additional stabilizing agent.

In a preferred embodiment, the liquid base of the foaming composition may further comprise an effective amount of at least one suitable disinfecting agent.

For the purposes of the invention, the disinfecting agent may be selected from the common topical disinfectants, provided they are compatible with the other ingredients of the foaming composition and, for instance, it may be selected from quaternary ammonium salts, disinfectants of the phenolic type, chlorexidine, and the like.

Preferably, the quantity of the disinfecting agent is comprised between 0.01 and 1% by weight based on the total weight of the liquid base.

In an embodiment of the invention, especially suitable in those cases where a cesspool is provided downstream of the water closet, the foaming composition may be so formulated as to contribute to the preservation and growth of the biomass present in the cesspool.

In this embodiment, the liquid base of the foaming composition further comprises an effective amount of a suitable substrate adapted to support the metabolic functions of the biomass present in said cesspool.

For the purposes of the invention, the liquid base of the foaming composition comprises in this case from 0.1 to 5% by weight and, still more preferably, from 0.2 to 1% by weight of said substrate based on the total weight of the liquid base.

Preferably, said substrate is selected from urea and from at least one amino acid selected from L-phenylalanine, L-arginine, L-aspartic acid, and mixtures thereof.

In this embodiment and if a disinfecting agent is also present, the amount of the latter agent is preferably and advantageously such that a topical action in the water closet is carried out without inducing significant detrimental effects on the vitality of the biomass present in the cesspool.

In a preferred embodiment, the liquid base of the foaming composition may further comprise an effective amount of at least one perfuming substance soluble in the solvent employed in the formulation of the liquid base.

For the purposes of the invention, said perfuming substance is selected from the fragrances commonly used in the field and is present in the liquid base of the foaming composition in a quantity ranging from 0.1 to 3% by weight based on the total weight of the liquid base.

Finally, the foaming composition of the invention may comprise additional additives in smaller amounts, such as for instance antioxidants, corrosion inhibitors, etc., whose amounts may be easily determined by the man skilled in the art according to the specific formulation requirements.

In order to cause the formation of a foam when the composition is dispensed, the foaming composition of the invention comprises a quantity of a gaseous propellant which may range from 5 to 30 parts by weight based on 100 parts of the composition.

As a consequence, the weight ratio between the gaseous propellant and the liquid base may range between 5:95 and 30:70.

Such ratios may be obtained by dosing in a manner known *per se* in a suitable pressurized container, for instance an aerosol bomb, the gaseous propellant and the liquid base previously obtained by mixing the various ingredients.

From the experiments carried out, the Applicant has found that in order to obtain an optimal duration of a foam having the structural and mechanical features required, the weight ratio of the gaseous propellant to the liquid base should preferably be comprised between 20:80 and 25:75.

Preferably, furthermore, the gaseous propellant is an hydrocarbon selected from propane, butane, and mixtures thereof, and still more preferably it is essentially constituted by a gaseous mixture of butane and propane.

In this case, the best results have been obtained when the weight ratio of butane to propane was equal to 3:2.

Additional features and advantages of the invention will become more readily apparent from the following description of some embodiments of the invention, given hereinbelow by way of non limiting indication.

In all the following examples, the compositions will be defined by indicating the parts by weight of each component, unless otherwise specified.

### EXAMPLE 1

By means of conventional mixing operations known *per se,* a liquid base was prepared having the composition shown in the following Table I (data in % by weight based on the total weight of the liquid base).

The following ingredients were employed:
- demineralized water;
- nonionic ethoxylated surfactant: a mixture of polyoxyethylene(20)cetyl alcohol commercially available as Brij® 58 (supplied by ICI SURFACTANTS, Middlesborough, Great Britain) and polyoxyethylene(4)lauryl alcohol commercially available as Brij® 30 (supplied by ICI SURFACTANTS);
- foam stabilizing agent: polyoxyethylene glycol having a molecular weight comprised between 5400 and 6600 or PEG 6000;
- disinfecting agent: 30% aqueous solution of bis(3-aminopropyl)dodecyl amine, commercially available as LONZABAC 12.30 (supplied by LONZA, Basle, CH);
- substrate for the support of the metabolic functions of biomass: mixture of L-phenyl alanine, L-arginine, L-aspartic acid;
- perfuming substance conventional *per se;*
- corrosion inhibitor: heptanoyldiethanolamine commercially available as CRODINHIB GM 5 (supplied by CRODA ITALIANA S.p.A., Pavia, Italy).

Starting from said base, a foaming composition according to the invention was obtained by dosing in a pressurized container of an aerosol bomb 80 parts by weight of liquid base and 20 parts by weight of a gaseous propellant constituted by a mixture of butane and propane in a 3:2 weight ratio.

### EXAMPLES 2-3

By means of conventional mixing operations known *per se,* two liquid bases were prepared having the compositions shown in Table I (data in % by weight based on the total weight of the liquid base).

With the same gaseous propellant of the preceding Example 1, two additional foaming compositions according to the invention were prepared by dosing in a pressurized container of an aerosol bomb 75 parts by weight of liquid base and 25 parts by weight of propellant (Example 2), and 80 parts by weight of liquid base and 20 parts by weight of propellant (Example 3).

### EXAMPLE 4

### (Determination of the physical-mechanical properties of the foam)

The mechanical and structural features of the foams obtainable by dispensing the foaming compositions of the preceding Examples 1-3 were evaluated utilizing the following protocol.

In a transparent graduated cylinder with 50 ml marks and having a diameter of 11 cm and a height of 13 cm, 8 g of foam were dispensed, after agitation of the aerosol bomb including the foaming composition to be tested.

During the introduction of the foam in the container, the foam dispensing means were suspended at 10 cm from the top opening of the container and the dispensing step was a continuous operation carried out until the above amount in weight of foam had been introduced, as determined by placing the container on a scale.

After the expansion of the foam, the height reached was measured so as to calculate its volume, and the mean diameter of the air bubbles was obtained by means of a calibrated grid, utilizing a 1:1 scale photograph of the foam.

The bubbles were then classified as follows:
- small bubbles: mean diameter < 1 mm;
- medium bubbles: mean diameter between 1 and 2 mm;
- large bubbles: mean diameter > 2 mm,

The results of the tests carried out (average values out of 3 experiments) are shown in the following Table II.

In all cases a compact, creamy, small-bubble foam was obtained having a foam reduction time depending on the ratio between the liquid base and the gaseous propellant. The best results were obtained with a 80:20 ratio between the liquid base and the gaseous propellant.

Subsequent tests carried out in bowl water closets and floor-latrines confirmed the effective barrier and perfuming effect against unpleasant odors and the lubricating effect against feces residues provided by the foam of the invention, when the same is dispensed on the free surface of t metabolic functions he stagnant water and/or on the water closet walls.

Clearly, a man skilled in the art may introduce modifications and variants to the invention described hereinabove, in order to satisfy specific and contingent application requirements, modifications and variants which however fall within the scope as defined by the appended claims.

**Table I**

| Ingredient | Ex. 1 | Ex. 2 | Ex. 3 |
|---|---|---|---|
| Demineralized water | balance to 100 | balance to 100 | balance to 100 |
| PEG 6000 | 5 | 8 | 6.5 |
| BRIJ 58 | 0.8 | 1.5 | 1 |
| BRIJ 30 | 0.5 | 0.7 | 0.8 |
| Perfume | 0.1 | 0.2 | 0.5 |
| LONZABAC 12.30 | 0.3 | 0.4 | 0.2 |
| L-phenylalanine | 0.13 | 0.13 | 0.13 |
| L-arginine | 0.13 | 0.13 | 0.13 |
| L-aspartic acid | 0.13 | 0.13 | 0.13 |
| CRODINHIB GM5 | 0.9 | 0.9 | 0.9 |

**Table II**

| Example | Time after dispensing (min) | | | | | Foam type |
|---|---|---|---|---|---|---|
| | 0 | 10 | 30 | 60 | 120 | |
| | Volume after foam dispensing (ml) | | | | | |
| 1 | 700 | 700 | 650 | 400 | 200 | Small bubbles |
| 2 | 600 | 600 | 200 | 50 | - | Small bubbles |
| 3 | 500 | 500 | 500 | 300 | 200 | Small bubbles |
| | Foam volume/starting volume* 100 | | | | | |
| 1 | 100 | 100 | 92.8 | 57.1 | 28.6 | Small bubbles |
| 2 | 100 | 100 | 33.3 | 8.3 | 0 | Small bubbles |
| 3 | 100 | 100 | 100 | 60 | 40 | Small bubbles |

## Claims

1. A method for reducing the emission of unpleasant odors from a water closet comprising the step of dispensing within the water closet a compact and creamy foam, the foam being such that by dispensing 8 g thereof in a cylindrical container having a diameter of 11 cm, the foam is essentially constituted by bubbles having a maximum diameter of 1 mm and a substantially constant volume for at least 10 minutes.

2. Method according to claim 1, wherein said foam is dispensed on the stagnant water present in the water closet, and optionally on the internal walls thereof near the free surface of the stagnant water.

3. Method according to claim 1, wherein said foam is dispensed on a closing door of a discharge opening of the water closet, and optionally on the internal walls thereof near said door.

4. A foaming composition adapted to generate a foam capable of reducing the emission of unpleasant odors from a water closet, comprising:
a) from 70 to 95% by weight of a liquid base including:
a1) from 50 to 90% by weight based on the total weigh of the liquid base of a suitable solvent;
a2) from 0.2 to 5% by weight based on the total weigh of the liquid base of a nonionic ethoxylated surfactant selected from ethoxylated alcohols having the formula
CH₃(CH₂)ₙCH₂(OCH₂CH₂)ₘOH
wherein n is an integer of from 10 to 16, m is an integer of from 2 to 24, salts of said alcohols soluble in said solvent, and mixtures thereof,
a3) from 2 to 12% by weight based on the total weight of the liquid base of a foam stabilizing agent,
the weight ratio between said nonionic ethoxylated surfactant and said foam stabilizing agent being comprised between 5:1 and 2:1, and
b) from 5 to 30% by weight of a gaseous propellant.

5. Foaming composition according to claim 4, wherein the solvent of the liquid base is constituted by demineralized water.

6. Foaming composition according to claim 4, characterized in that said nonionic ethoxylated surfactant is essentially constituted by a mixture of polyoxyethylene(20)cetyl alcohol and polyoxyethylene(4)lauryl alcohol.

7. Foaming composition according to claim 6, wherein said nonionic ethoxylated surfactant comprises:
- from 0.5 to 3% by weight of polyoxyethylene(20)cetyl alcohol,
- from 0.2 to 2% by weight ofpolyoxyethylene(4)lauryl alcohol.

8. Foaming composition according to claim 4, wherein said foam stabilizing agent is selected from amine alkyl oxides, coconut monoethanolamide, sodium lauryl succinate, polyoxyethyleneglycol, hydrocolloids, optionally functionalized with a quaternary ammonium group, acrylic and methacrylic polymers, and mixtures thereof.

9. Foaming composition according to claim 8, wherein said foam stabilizing agent is a polyethylene glycol having a molecular weight comprised between 5400 and 6600.

10. Foaming composition according to claim 4, wherein the weight ratio between said nonionic ethoxylated surfactant and said foam stabilizing agent is comprised between 3:1 and 4:1.

11. Foaming composition according to claim 4, wherein said liquid base further comprises an effective amount of at least one suitable disinfecting agent.

12. Foaming composition according to claim 4, wherein said liquid base further comprises an effective amount of a suitable substrate adapted to support the metabolic functions of the biomass present in a cesspool.

13. Foaming composition according to claim 12, wherein said substrate comprises at least one amino acid selected from L-phenylalanine, L-arginine, L-aspartic acid, and mixtures thereof.

14. Foaming composition according to claim 4, wherein said liquid base further comprises an effective amount of at least one perfuming substance soluble in said solvent.

15. Foaming composition according to claim 4, wherein the weight ratio between the gaseous propellant and the liquid base is comprised between 20:80 and 25:75.

16. Foaming composition according to claim 4, wherein the gaseous propellant is a hydrocarbon comprising propane, butane, and mixtures thereof.

17. Foaming composition according to claim 16, wherein the gaseous propellant is essentially constituted by a gaseous mixture of propane and butane.

18. An aerosol bomb comprising:
- a pressurized container including a dosed amount of a foaming composition according to anyone of claims 4-17,
- dispensing means for delivering a foam from said foaming composition.
